(19) **Europäisches Patentamt**
**European Patent Office**
**Office européen des brevets**

(11) **EP 3 846 007 A1**

(12) **EUROPEAN PATENT APPLICATION**

(43) Date of publication:
**07.07.2021 Bulletin 2021/27**

(21) Application number: **20208081.8**

(22) Date of filing: **17.11.2020**

(51) Int Cl.:
*G06F 3/01* (2006.01)  *G06F 3/0481* (2013.01)
*A61H 1/02* (2006.01)  *A61H 3/00* (2006.01)
*B25J 9/00* (2006.01)  *A63B 24/00* (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
**KH MA MD TN**

(30) Priority: **30.12.2019 KR 20190177938**

(71) Applicant: **Samsung Electronics Co., Ltd.**
**Suwon-si 16677 (KR)**

(72) Inventors:
- **Hyung, Seungyong**
 **16677 Gyeonggi-do (KR)**
- **Kim, Kyungrock**
 **16677 Gyeonggi-do (KR)**
- **Roh, Segon**
 **16677 Gyeonggi-do (KR)**
- **Lim, Bokman**
 **16677 Gyeonggi-do (KR)**

(74) Representative: **Arnold & Siedsma**
**Bezuidenhoutseweg 57**
**2594 AC The Hague (NL)**

(54) **WEARABLE DEVICE WITH FORCE FEEDBACK AND OPERATION METHOD THEREOF**

(57) A wearable device receives control information for controlling the wearable device from a virtual reality (VR) device, determines force output information corresponding to a walking environment of a user in a VR based on the received control information, and outputs force feedback based on the determined force output information.

EP 3 846 007 A1

**Description**

BACKGROUND

1. Field

At least one example embodiment relates to a wearable device and an operation method of the wearable device.

2. Description of the Related Art

There is a growing attention on a walking assist device that assists a user in walking. In addition, an exercise assist device is being developed to strengthen the muscular strength of a user.

A walking assistance training or an exercise assistance training may be performed in virtual reality (VR).

SUMMARY

Some example embodiments relate to a method of operating a wearable device.

In some example embodiments, the method includes receiving, from a virtual reality (VR) device, control information associated with controlling the wearable device; determining force output information corresponding to a walking environment of a user in a VR environment based on the control information; and outputting force feedback to the user based on the force output information.

In some example embodiments, the control information includes at least one of a gradient of a walking path in the VR environment or a parameter set for the walking environment.

In some example embodiments, the parameter includes at least one of a gain associated with a magnitude and a direction of the force feedback, or a delay associated with an output timing of the force feedback.

In some example embodiments, the receiving of the control information comprises: receiving a gradient from the VR device, when the walking environment includes the gradient.

In some example embodiments, the determining of the force output information comprises: determining the force output information based on the gradient such that the force feedback is a resistance force, when the gradient has a positive value; and determining the force output information based on the gradient such that the force feedback is an assistance force, when the gradient has a negative value.

In some example embodiments, the determining of the force output information comprises: determining a gain based on the gradient; and determining the force output information based on the gain.

In some example embodiments, the determining of the gain comprises: determining the gain as a negative gain, when the gradient has a positive value; and determining the gain as a positive gain, when the gradient has a negative value.

In some example embodiments, the receiving of the control information comprises: receiving parameters set for a water walking environment from the VR device when the walking environment is the water walking environment.

In some example embodiments, the determining of the force output information comprises: determining the force output information based on the parameters, the parameters including a gain that allows the force feedback to be a resistance force and a delay that delays an output timing of the force feedback.

In some example embodiments, the method further includes transmitting walking information to the VR device.

Some example embodiments relate to a wearable device.

In some example embodiments, the wearable device includes a communicator configured to communicate with a virtual reality (VR) device; a driver configured to output a force feedback to a user; and a controller configured to, receive control information associated with controlling the wearable device from the VR device through the communicator, determine force output information corresponding to a walking environment of the user in a VR environment based on the control information, and control the driver to output the force feedback based on the force output information.

In some example embodiments, the control information includes at least one of a gradient associated with a walking path in the VR environment or a parameter set for the walking environment.

In some example embodiments, the parameter includes at least one of a gain associated with a magnitude and a direction of the force feedback or a delay associated with an output timing of the force feedback.

In some example embodiments, the controller is configured to receive a gradient from the VR device through the communicator, when the walking environment includes the gradient.

In some example embodiments, the controller is configured to, determine the force output information based on the gradient such that the force feedback is a resistance force, when the gradient has a positive value, and determine the force output information based on the gradient such that the force feedback is an assistance force, when the gradient has a negative value.

In some example embodiments, the controller is configured to, determine a gain based on the gradient, and determine the force output information based on the gain.

In some example embodiments, the controller is configured to, determine the gain as a negative gain, when the gradient has a positive value, and determine the gain as a positive gain, when the gradient has a negative value.

In some example embodiments, the controller is configured to receive parameters set for a water walking environment from the VR device through the communicator, when the walking environment is the water walking environment.

In some example embodiments, the controller is configured to determine the force output information based on the parameters, the parameters including a gain that allows the force feedback to be a resistance force and a delay that delays an output timing of the force feedback.

In some example embodiments, the controller is configured to transmit walking information to the VR device through the communicator.

Additional aspects of example embodiments will be set forth in part in the description which follows and, in part, will be apparent from the description, or may be learned by practice of the disclosure.

BRIEF DESCRIPTION OF THE DRAWINGS

These and/or other aspects will become apparent and more readily appreciated from the following description of example embodiments, taken in conjunction with the accompanying drawings of which:

> FIGS. 1 through 3 are diagrams illustrating an example of a wearable device according to at least one example embodiment;
> FIGS. 4 through 8 are diagrams illustrating examples of operations of a wearable device and a virtual reality (VR) device according to at least one example embodiment;
> FIG. 9 is a diagram illustrating an example of a configuration of a wearable device according to at least one example embodiment; and
> FIG. 10 is a flowchart illustrating an example of an operation method of a wearable device according to at least one example embodiment.

DETAILED DESCRIPTION

Hereinafter, some example embodiments will be described in detail with reference to the accompanying drawings. Regarding the reference numerals assigned to the elements in the drawings, it should be noted that the same elements will be designated by the same reference numerals, wherever possible, even though they are shown in different drawings. Also, in the description of embodiments, detailed description of well-known related structures or functions will be omitted when it is deemed that such description will cause ambiguous interpretation of the present disclosure.

It should be understood, however, that there is no intent to limit this disclosure to the particular example embodiments disclosed. On the contrary, example embodiments are to cover all modifications, equivalents, and alternatives falling within the scope of the example embodiments. Like numbers refer to like elements throughout the description of the figures.

In addition, terms such as first, second, A, B, (a), (b), and the like may be used herein to describe components. Each of these terminologies is not used to define an essence, order or sequence of a corresponding component but used merely to distinguish the corresponding component from other component(s). It should be noted that if it is described in the specification that one component is "connected," "coupled," or "joined" to another component, a third component may be "connected," "coupled," and "joined" between the first and second components, although the first component may be directly connected, coupled or joined to the second component.

The terminology used herein is for the purpose of describing particular embodiments only and is not intended to be limiting. As used herein, the singular forms "a," "an," and "the," are intended to include the plural forms as well, unless the context clearly indicates otherwise. It will be further understood that the terms "comprises," "comprising," "includes," and/or "including," when used herein, specify the presence of stated features, integers, steps, operations, elements, and/or components, but do not preclude the presence or addition of one or more other features, integers, steps, operations, elements, components, and/or groups thereof.

It should also be noted that in some alternative implementations, the functions/acts noted may occur out of the order noted in the figures. For example, two figures shown in succession may in fact be executed substantially concurrently or may sometimes be executed in the reverse order, depending upon the functionality/acts involved.

Unless otherwise defined, all terms, including technical and scientific terms, used herein have the same meaning as commonly understood by one of ordinary skill in the art to which the disclosure of this application pertains. Terms, such as those defined in commonly used dictionaries, are to be interpreted as having a meaning that is consistent with their meaning in the context of the relevant art, and are not to be interpreted in an idealized or overly formal sense unless expressly so defined herein.

Also, in the description of example embodiments, detailed description of structures or functions that are thereby known after an understanding of the disclosure of the present application will be omitted when it is deemed that such description will cause ambiguous interpretation of the example embodiments.

Various example embodiments will now be described more fully with reference to the accompanying drawings in which some example embodiments are shown. In the drawings, the thicknesses of layers and regions are exaggerated for clarity.

Hereinafter, example embodiments will be described in detail with reference to the accompanying drawings, and like reference numerals in the drawings refer to like elements throughout.

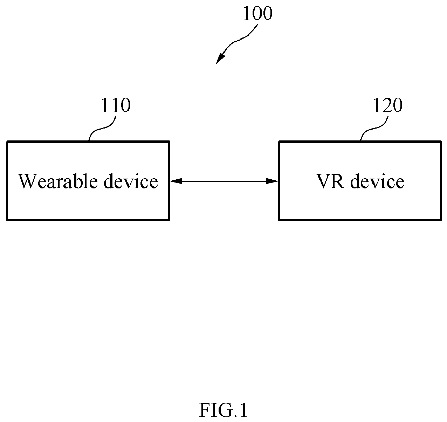

FIGS. 1 through 3 are diagrams illustrating an example of a wearable device according to at least one example embodiment.

Referring to FIG. 1, a wearable system 100 includes a wearable device 110 and a virtual reality (VR) device 120.

The wearable device 110 may be paired with the VR device 120. For example, the wearable device 110 may be paired with the VR device 120 through a wireless communication link, for example, Bluetooth. According to another example, the wearable device 120 and the VR device 120 may be connected to each other through wired communication, for example, through a cable.

The VR device 120 may display a VR environment or situation on a display of the VR device 120. A user may walk with the wearable device 110 and the VR device 120 on, and the user may feel as if he/she walks in such an environment or situation represented by the VR environment or situation.

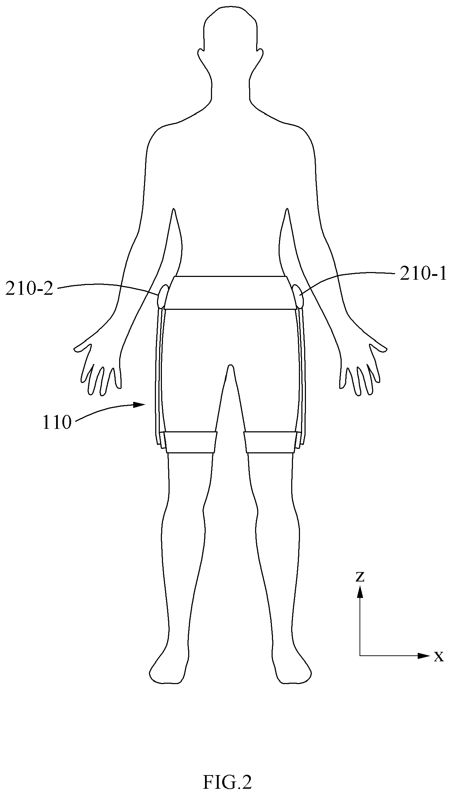

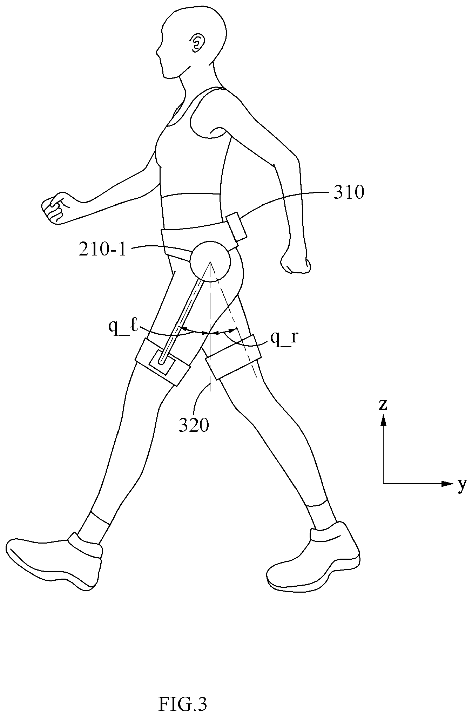

The wearable device 110 may be provided as a hip-type device that is worn on or around a hip joint or a thigh of the user, an ankle-type device that is worn on or around an ankle of the user, or a knee-type device that is worn on or around a knee of the user. However, types of the wearable device 110 are not limited to the foregoing example types. The wearable device 110 that is provided in a hip type is illustrated in FIGS. 2 and 3.

Referring to FIGS. 2 and 3, drivers 210-1 and 210-2 of the wearable device 110 may be disposed around a hip joint of a user, and a controller 310 of the wearable device 110 may be disposed around a waist of the user. However, positions of the drivers 210-1 and 210-2 and the controller 310 are not limited to the example positions illustrated in FIGS. 2 and 3.

The wearable device 110 may measure or sense a left hip joint angle $q\_l$ of the user and a right hip joint angle $q\_r$ of the user. For example, the wearable device 110 may measure or sense the left hip joint angle $q\_l$ through a left encoder, and the right hip joint angle $q\_r$ through a right encoder. Referring to FIG. 3, a left leg of the user is ahead of a reference line 320, and thus the left hip joint angle $q\_l$ may be a negative value. In addition, a right leg of the user is behind the reference line 320, and thus the right hip joint angle $q\_r$ may be a positive value. According to another example, the right hip joint angle $q\_r$ may be a negative value when the right leg is ahead **of** the reference line 320, and the left hip joint angle $q\_l$ may be a positive value when the left leg is behind the reference line 320.

The wearable device 110 may determine force output information $\tau(t)$ based on the left hip joint angle $q\_l$, the right hip joint angle $q\_r$, a gain $\kappa$, and a delay $\Delta t$. The wearable device 110 may then output force feedback based on the determined force output information $\tau(t)$. For example, the wearable device 110 may determine the force output information $\tau(t)$ as represented by Equation 1 below.

Equation 1

$$y = sin(q\_r) - sin(q\_l)$$

$$\tau(t) = \kappa y(t - \Delta t)$$

The gain $\kappa$ may be a parameter indicating a magnitude and a direction of the force feedback. When a magnitude of the gain $\kappa$ increases, greater force feedback may be output. For example, when the gain $\kappa$ is a negative value, a resistance force may be output as the force feedback. In contrast, when the gain $\kappa$ is a positive value, an assistance force may be output as the force feedback. The delay $\Delta t$ may be a parameter associated with an output timing of the force feedback.

According to an example, the wearable device 110 may output or provide, to the user, the force feedback that is suitable for a VR environment or situation by interworking with the VR device 120 and determining the force output information $\tau(t)$. Although to be described hereinafter, for example, when a user walks up an ascending road in VR, the wearable device 110 may determine force output information corresponding to such a VR environment or situation, and output force feedback to the user based on the determined force output information. For another example, when a user walks in the water in VR, the wearable device 110 may determine force output information corresponding to such a VR environment or situation, and output force feedback to the user based on the determined force output information.

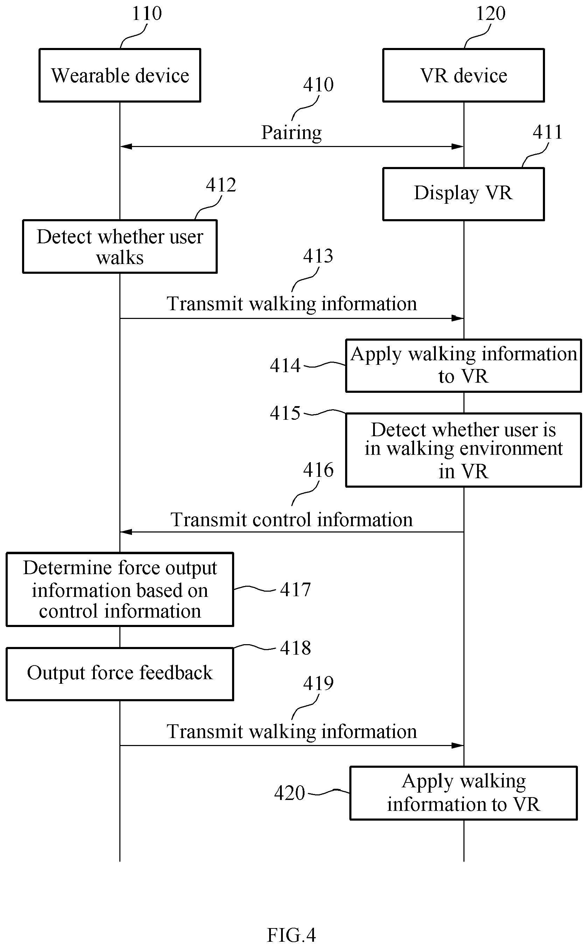

FIGS. 4 through 8 are diagrams illustrating examples of operations of a wearable device and a VR device according to at least one example embodiment.

Referring to FIG. 4, in operation 410, the wearable device 110 is paired with the VR device 120. For example, the wearable device 110 and the VR device 120 may establish a wireless communication link, for example, a Bluetooth communication link, therebetween. However, examples are not limited to the foregoing example, and the wearable device 110 and the VR device 120 may be connected to each other through a cable.

In operation 411, the VR device 120 displays a VR on a display of the VR device 120.

In operation 412, the wearable device 110 detects whether a user walks. For example, when at least one of a left hip joint angle $q\_l$ or a right hip joint angle $q\_r$ is started being sensed, the wearable device 110 may determine that the user walks.

In operation 413, when the wearable device 110 detects that the user walks, the wearable device 110 obtains walking information and transmits the obtained walking information to the VR device 120. The walking information may include at least one of walking speed information or pose information.

In operation 414, the VR device 120 applies, to the VR, the walking information received from the wearable device 110. Thus, the user may visually experience a feeling as if he/she walks in the VR. Further, as discussed below, the wearable device 110 may provide the user with a physical experience corresponding to the walking environment in the VR

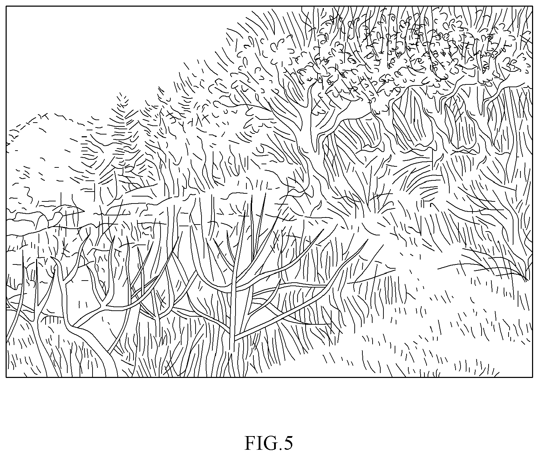

In operation 415, the VR device 120 detects whether the user is in a distinguishing walking environment in the VR. For example, the VR device 120 may determine whether the user is in such a walking environment based on a location of the user in the VR. The walking environment may be determined in advance. The walking environment may include, for example, a walking environment with a gradient (e.g., an ascending road, a descending road, and a staircase) and/or a water walking environment. However, examples of the walking environment are not limited to the foregoing examples. The examples of the walking environment will be described hereinafter with reference to FIGS. 5 through 8.

In operation 416, when the user is in one of the distinguishing the walking environments in the VR, the VR device 120 transmits, to the wearable device 110, control information for controlling the wearable device 110. The VR device 120 may transmit, to the wearable device 110, the control information such that the wearable device 110 may output force feedback corresponding to the walking environment. The control information may include at least one of a gradient of a walking path in the VR or a parameter. The parameter may include at least one of a gain $\kappa$ or a delay $\Delta t$.

In operation 417, the wearable device 110 determines force output information based on the control information received from the VR device 120. For example, the wearable device 110 may determine the force output information using Equation 1.

In operation 418, the wearable device 110 outputs force feedback based on the force output information determined in operation 417. For example, when the wearable device 110 receives a gradient of a walking path in the VR from the VR device 120, the wearable device 110 may determine a gain $\kappa$ based on the received gradient. In this example, when the received gradient is a positive value, the wearable device 110 may determine a negative gain -$\kappa$, and determine force output information based on the determined negative gain -$\kappa$. The wearable device 110 may then output force feedback based on the determined force output information. The force feedback to be output may correspond to a resistance force that gives the user resistance while the user is walking. When the user walks up an actual ascending road in a reality, the user may have a heavy or uncomfortable feeling in his/her leg. When the user walks up an ascending road in a VR, the wearable device 110 may output force feedback corresponding to a resistance force such that the user may experience the feeling the user has when the user walks up the actual ascending road in the reality. That is, when the user walks up the ascending road in the VR, the user may receive resistance from the wearable device 110. Thus, the user may experience, in the VR, the feeling the user has when the user walks up the actual ascending road in the reality.

In operation 419, the wearable device 110 obtains walking information of the user to which the force feedback is provided, and transmits the obtained walking information to the VR device 120. The walking information may include at least one of walking speed information or pose information.

In operation 420, the VR device 120 applies, to the VR, the walking information received from the wearable device 110. Thus, for example, the walking information indicating that the user walks at a reduced speed and/or the user loses his/her posture may be applied to or represented in the VR.

FIG. 5 illustrates an example of an ascending road in a VR

The VR device 120 may display an ascending road on a display of the VR device 120 as illustrated in FIG. 5.

When a user walks, the VR device 120 may output, to the display, visual feedback such that the user feels as if the user walks up the ascending road. Thus, the user may feel as if the user walks up the ascending road in a visual way in a VR.

The user may experience a load applied to a leg of the user when the user walks up an actual ascending road in a reality. To represent in the VR such a load the user may experience, the wearable device 110 may output force feedback that is a resistance force. For example, when the user enters the illustrated ascending road in the VR, the VR device 120 may transmit a gradient of the ascending road to the wearable device 110. In this example, the gradient may be a positive value. The wearable device 110 may then determine a negative gain -$\kappa$ based on the gradient of the ascending road. For example, when the wearable device 110 receives a gradient g_1 from the VR device 120, the wearable device 110 may refer to Table 1 below and find a gain $\kappa$g_1 mapped to the received gradient g_1.

Table 1

| Gradient | Gain |
|---|---|
| g_n (maximum) | $K_{g\_n}$ |
| g_1 | $K_{g\_1}$ |
| 0 | $K_0$ |
| -g_1 | $K_{-g\_1}$ |
|  |  |
| -g_8 | $K_{-g\_8}$ |
| ... | ... |
| -g_n | $K_{-g\_n}$ |

Referring to Table 1 above, $\kappa$g_1 through $\kappa$g_n are negative values. Herein, a magnitude of an absolute value may increase in order starting from $\kappa$g_1 towards

$\kappa$g_n. That is, when a gradient of an ascending road increases, a magnitude of an absolute value of a negative gain may increase.

In addition, $\kappa$0 through $\kappa$-g_n are positive values. Herein, a magnitude may increase in order starting from $\kappa$0 towards $\kappa$-g_n. That is, when a gradient of a descending road increases, a magnitude of a positive gain may increase.

According to an example, the wearable device 110 may receive, from the VR device 120, a gain mapped to a gradient. For example, the VR device 120 may store Table 1. In this example, when the gradient of the ascending road illustrated in FIG. 5 is g_1, the VR device 120 may transmit, to the wearable device 110, $\kappa$g_1 mapped to g_1.

The wearable device 110 may determine force output information using the negative gain $\kappa$g_1, a delay $\Delta$t, and Equation 1 described above. The delay $\Delta$t may be 0, but not limited thereto.

The wearable device 110 may output force feedback that is a resistance force based on the determined force output information. Thus, the user may experience, in the VR, a load applied to a leg of the user, which is similar to what the user experiences when the user walks up an actual ascending road in a reality.

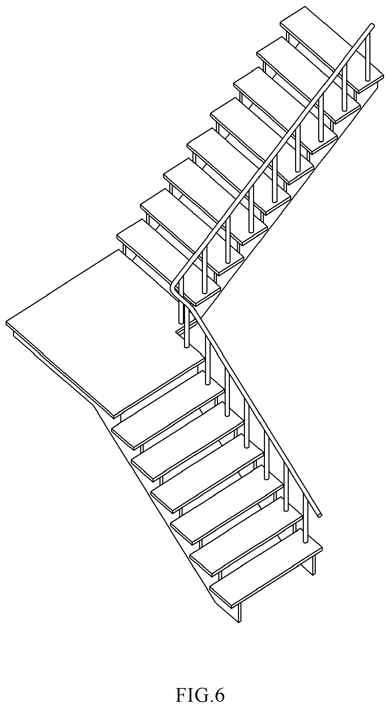

FIG. 6 illustrates an example of a staircase in a VR.

The VR device 120 may display a staircase on a display of the VR device 120 as illustrated in FIG. 6.

When a user walks, the VR device 120 may output, to the display, visual feedback such that the user feels as if the user walks down the stairs. Thus, the user may feel as if the user walks down or up the stairs in a visual way in a VR.

For example, when the user enters the illustrated stairs in the VR from the top of the staircase, the VR device 120 may transmit a gradient of the stairs to the wearable device 110. In this example, the gradient may be a negative value. The wearable device 110 may then determine a positive gain $\kappa$ based on the gradient of the stairs. For example, when the wearable device 110 receives a gradient -g_8 from the VR device 120, the wearable device 110 may refer to Table 1 above and find a gain $\kappa$-g_8 mapped to the received gradient -g_8. According to another example, the wearable device 110 may receive the gain $\kappa$-g_8 from the VR device 120.

The wearable device 110 may determine force output information using the gain $\kappa$-g_8, a delay $\Delta$t, and Equation 1 described above. The delay $\Delta$t may be 0, but not limited thereto.

The wearable device 110 may output force feedback that is an assistance force based on the determined force output information. When the user walks down actual stairs in an actual reality, the user may receive a slight force due to gravity. Similarly, when the user walks down the stairs in the VR, the wearable device 110 may output, to the user, force feedback that is an assistance force. Thus, the user may receive the assistance force in the VR, which is similar to what the user experiences when the user walks down the actual stairs in the reality.

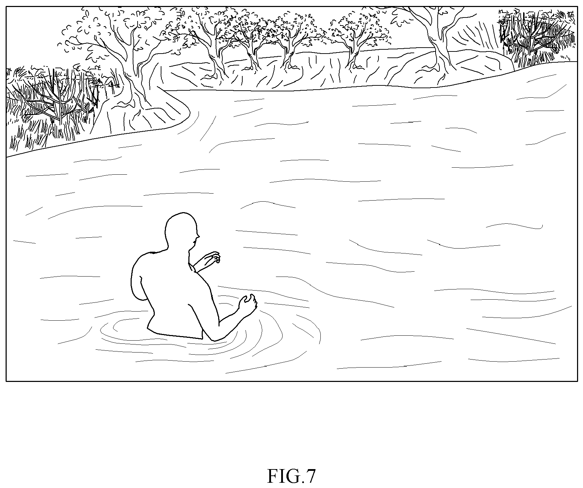

FIG. 7 illustrates an example of a water walking environment in a VR.

The VR device 120 may display a water walking environment on a display of the VR device 120 as illustrated in FIG. 7.

When a user walks, the VR device 120 may output, to the display, visual feedback such that the user feels as if the user walks in the water. Thus, the user may visually feel as if the user walks in the water in a visual way in a VR.

Further, the VR device 120 may transmit, to the wearable device 110, a negative gain - $\kappa$water and a delay $\Delta$twater that delays an output timing of force feedback.

The wearable device 110 may determine force output information corresponding to the water walking environment using the negative gain -$\kappa$water, the delay $\Delta$twater, and Equation 1 described above.

The wearable device 110 may output force feedback that is a resistance force based on the determined force output information. When the user walks in the water in an actual reality, the user may receive resistance by the water. Similarly, when the user walks in the water in the VR, the wearable device 110 may output, to the user, force feedback that is a resistance force. Thus, the user may receive the resistance force in the VR, which is similar to what the user experiences when the user walks in the water in the reality.

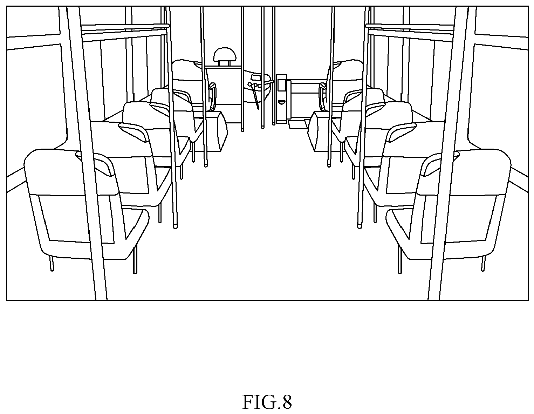

FIG. 8 illustrates an example of an in-bus environment in a VR.

The VR device 120 may display an internal environment of a bus on a display of the VR device 120 as illustrated in FIG. 8.

The VR device 120 may output, to the display, visual feedback such that a user may visually experience a feeling as if the user moves by bus. Thus, the user may visually experience a feeling as if the user moves by bus in a visual way in a VR.

Further, when the user embarks on a bus in a VR, the VR device 120 may notify the wearable device 110 that the user embarks on the bus in the VR. The wearable device 120 may then output a vibration corresponding to such a situation in which the user moves by bus in the VR. For example, the VR device 120 may instruct the wearable device 120 to vary the gain and/or the delay to provide a vibration to the user.

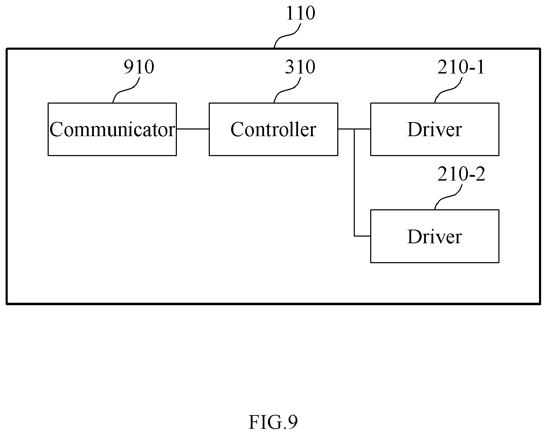

FIG. 9 is a diagram illustrating an example of a configuration of a wearable device according to at least one example embodiment.

Referring to FIG. 9, the wearable device 110 includes a communicator 910, the controller 310, and the drivers 210-1 and 210-2. According to other examples, the wearable device 110 may include a single driver, or three or more drivers.

The communicator 910 may connect the wearable device 110 and the VR device 120. For example, the communicator 910 may connect the wearable device 110 and the VR device 120 through a wireless communication link (e.g., Bluetooth) or a cable.

At least one of the drivers 210-1 and 210-2 may output force feedback.

The controller 310 may include processing circuitry including, but is not limited to, a central processing unit (CPU), an arithmetic logic unit (ALU), a digital signal processor, a microcomputer, a field programmable gate array (FPGA), a programmable logic unit, a microprocessor, application-specific integrated circuit (ASIC), etc. The processing circuitry may be special purpose processing circuitry that configures the wearable device 1 to determine the force output information $\tau(t)$ and provide force feedback to the user based on the force output information $\tau(t)$ that is suitable for a VR environment or situation by interworking with the VR device 120. Therefore, the special purpose controller 310 may improve the functioning of the wearable device 110 and/or the wearable system 100 by providing the user with a physical experience corresponding to walking in the VR environment generated by the VR device 120.

The controller 310 may receive control information for controlling the wearable device 110 from the VR device 120 through the communicator 910. The control information may include, for example, at least one of a gradient of a walking path in a VR, and a parameter set for a walking environment in the VR. For example, when the walking environment is a walking environment with a gradient, for example, the ascending road illustrated in FIG. 5 or the staircase illustrated in FIG. 6, the controller 310 may receive the gradient from the VR device 120. For another example, when a user enters a water walking environment in a VR as illustrated in FIG. 7, the controller 310 may receive, from the VR device 120, a negative gain $\kappa$water and a delay $\Delta$twater that are set for the water walking environment.

The controller 310 may determine force output information corresponding to the walking environment of the user in the VR based on the received control information. For example, when the gradient received from the VR device 120 is a positive value, the controller 310 may determine the force output information such that the force feedback is a resistance force. In contrast, when the gradient received from the VR device 120 is a negative value, the controller 310 may determine the force output information such that the force feedback is an assistance force.

The controller 310 may control at least one of the drivers 210-1 and 210-2 based on the determined force output information.

Although not illustrated in FIG. 9, the wearable device 110 may include an inertial measurement unit (IMU) sensor. The wearable device 110 may obtain pose information based on a sensing result of the IMU sensor. For example, the wearable device 110 may sense, using the IMU sensor, at least one of an angle associated with a movement of a user in a roll direction or an angle associated with a movement in a pitch direction, and obtain pose information based on a result of the sensing.

For a more detailed description of the wearable device 110 described above with reference to FIG. 9, reference may be made to what has been described above with reference to FIGS. 1 through 8.

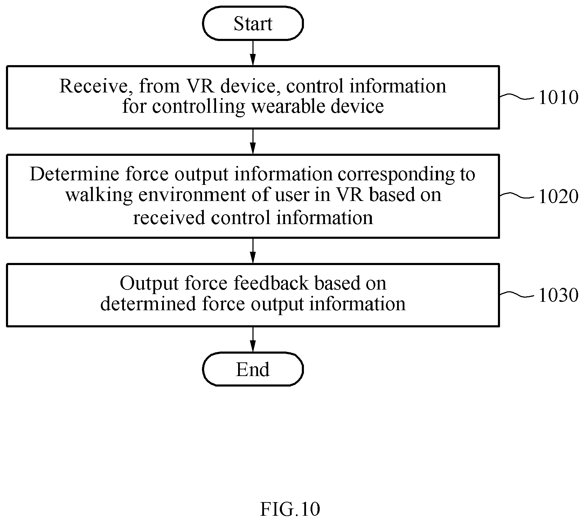

FIG. 10 is a flowchart illustrating an example of an operation method of a wearable device according to at least one example embodiment.

Referring to FIG. 10, in operation 1010, the wearable device 110 receives control information for controlling the wearable device 110 from the VR device 120.

In operation 1020, the wearable device 110 determines force output information corresponding to a walking environment of a user in a VR based on the received control information.

In operation 1030, the wearable device 110 outputs force feedback based on the determined force output information.

For a more detailed description of the operation method described above with reference to FIG. 10, reference may be made to what has been described above with reference to FIGS. 1 through 9.

According to an example, the wearable device 110 may be used as a joystick or a human machine interface (HMI) for a VR game by interworking with the VR device 120. The wearable device 110 may be used as a body joystick or an HMI for a VR game. For example, as described above, force feedback corresponding to a VR situation may be output by the wearable device 110. That is, the wearable device 110 may be used as an output means. In addition, walking information obtained by the wearable device 110 may be applied to a VR. That is, the wearable device 110 may be used as an input means.

In addition, the wearable device 110 and the VR device 120 may enable a user to do an exercise (e.g., rehabilitation exercise, correction exercise, and muscle strengthening exercise) through VR contents (e.g., VR games).

The units and/or modules described herein may be implemented using hardware components and software components. For example, the hardware components may include microphones, amplifiers, band-pass filters, audio to digital convertors, and processing devices. A processing device may be implemented using one or more hardware device configured to carry out and/or execute program code by performing arithmetical, logical, and input/output operations. The processing device(s) may include a processor, a controller and an arithmetic logic unit, a digital signal processor, a microcomputer, a field programmable array, a programmable logic unit, a microprocessor or any other device capable of responding to and executing instructions in a defined manner. The processing device may run an operating system (OS) and one or more software applications that run on the OS. The processing device also may access, store, manipulate, process, and create data in response to execution of the software. For purpose of simplicity, the description of a processing device is used as singular; however, one skilled in the art will appreciated that a processing device may include multiple processing elements and multiple types of processing elements. For example, a processing device may include multiple processors or a processor and a controller. In addition, different processing configurations are possible, such a parallel processors.

The software may include a computer program, a piece of code, an instruction, or some combination thereof, to independently or collectively instruct and/or configure the processing device to operate as desired, thereby transforming the processing device into a special purpose processor. Software and data may be embodied permanently or temporarily in any type of machine, component, physical or virtual equipment, computer storage medium or device, or in a propagated signal wave capable of providing instructions or data to or being interpreted by the processing device. The software also may be distributed over network coupled computer systems so that the software is stored and executed in a distributed fashion. The software and data may be stored by one or more non-transitory computer readable recording mediums.

The methods according to the above-described example embodiments may be recorded in non-transitory computer-readable media including program instructions to implement various operations of the above-described example embodiments. The media may also include, alone or in combination with the program instructions, data files, data structures, and the like. The program instructions recorded on the media may be those specially designed and constructed for the purposes of example embodiments, or they may be of the kind well-known and available to those having skill in the computer software arts. Examples of non-transitory computer-readable media include magnetic media such as hard disks, floppy disks, and magnetic tape; optical media such as CD-ROM discs, DVDs, and/or Blue-ray discs; magneto-optical media such as optical discs; and hardware devices that are specially configured to store and perform program instructions, such as read-only memory (ROM), random access memory (RAM), flash memory (e.g., USB flash drives, memory cards, memory sticks, etc.), and the like. Examples of program instructions include both machine code, such as produced by a compiler, and files containing higher level code that may be executed by the computer using an interpreter. The above-described devices may be configured to act as one or more software modules in order to perform the operations of the above-described example embodiments, or vice versa.

A number of example embodiments have been described above. Nevertheless, it should be understood that various modifications may be made to these example embodiments. For example, suitable results may be achieved if the described techniques are performed in a different order and/or if components in a described system, architecture, device, or circuit are combined in a different manner and/or replaced or supplemented by other components or their equivalents.

Accordingly, other implementations are within the scope of the following claims.

## Claims

1. A method of operating a wearable device, the method comprising:

   receiving, from a virtual reality (VR) device, control information associated with controlling the wearable device;
   determining force output information corresponding to a walking environment of a user in a VR environment based on the control information; and
   outputting force feedback to the user based on the force output information.

2. The method of claim 1, wherein
   the control information includes at least one of a gradient of a walking path in the VR environment or a parameter set for the walking environment, or
   the parameter includes at least one of a gain associated with a magnitude and a direction of the force feedback, or a delay associated with an output timing of the force feedback.

3. The method of claim 1 or 2, wherein
   the receiving of the control information includes receiving a gradient from the VR device, when the walking environment includes the gradient, or
   the determining of the force output information includes,
   determining the force output information based on the gradient such that the force feedback is a resistance force, when the gradient has a positive value; and
   determining the force output information based on the gradient such that the force feedback is an assistance force, when the gradient has a negative value.

4. The method of claim 3, wherein the determining of the force output information comprises:

   determining a gain based on the gradient; and
   determining the force output information based on the gain.

5. The method of claim 4, wherein the determining of the gain comprises:

   determining the gain as a negative gain, when the gradient has a positive value; and
   determining the gain as a positive gain, when the gradient has a negative value.

6. The method of any of the previous claims, wherein the receiving of the control information includes receiving parameters set for a water walking environment from the VR device when the walking environment is the water walking environment, or the determining of the force output information includes determining the force output information based on the parameters, the parameters including a gain that allows the force feedback to be a resistance force and a delay that delays an output timing of the force feedback.

7. The method of claim 1, further comprising: transmitting walking information to the VR device.

8. A wearable device comprising:

a communicator configured to communicate with a virtual reality (VR) device;
a driver configured to output a force feedback to a user; and
a controller configured to,
receive control information associated with controlling the wearable device from the VR device through the communicator,
determine force output information corresponding to a walking environment of the user in a VR environment based on the control information, and
control the driver to output the force feedback based on the force output information.

9. The wearable device of claim 8, wherein
the control information includes at least one of a gradient associated with a walking path in the VR environment or a parameter set for the walking environment, or
the parameter includes at least one of a gain associated with a magnitude and a direction of the force feedback or a delay associated with an output timing of the force feedback.

10. The wearable device of claim 9, wherein, the controller is configured to receive a gradient from the VR device through the communicator, when the walking environment includes the gradient.

11. The wearable device of claim 10, wherein
the controller is configured to determine the force output information based on the gradient such that the force feedback is a resistance force, when the gradient has a positive value, and determine the force output information based on the gradient such that the force feedback is an assistance force, when the gradient has a negative value, or
the controller is configured to determine a gain based on the gradient, and determine the force output information based on the gain.

12. The wearable device of claim 11, wherein the controller is configured to,
determine the gain as a negative gain, when the gradient has a positive value, and
determine the gain as a positive gain, when the gradient has a negative value.

13. The wearable device of any of claims 8-12, wherein,
the controller is configured to receive parameters set for a water walking environment from the VR device through the communicator, when the walking environment is the water walking environment, or
the controller is configured to determine the force output information based on the parameters, the parameters including a gain that allows the force feedback to be a resistance force and a delay that delays an output timing of the force feedback.

14. The wearable device of any of claims 8-13, wherein the controller is configured to transmit walking information to the VR device through the communicator.

100

110                                    120

| Wearable device | ←→ | VR device |

FIG.1

FIG.2

FIG.3

110

Wearable device

120

VR device

410

Pairing

411

Display VR

412

Detect whether user walks

413

Transmit walking information

414 — Apply walking information to VR

415 — Detect whether user is in walking environment in VR

416

Transmit control information

Determine force output information based on control information — 417

Output force feedback — 418

419

Transmit walking information

420 — Apply walking information to VR

FIG.4

FIG.5

FIG.6

FIG.7

FIG.8

110

910        310        210-1

| Communicator | Controller | Driver |

210-2

| Driver |

FIG.9

```
                      ╭─────────────╮
                      │    Start    │
                      ╰─────────────╯
                             │
                             ▼
   ┌─────────────────────────────────────────────┐
   │   Receive, from VR device, control information│
   │      for controlling wearable device         │──── 1010
   └─────────────────────────────────────────────┘
                             │
                             ▼
   ┌─────────────────────────────────────────────┐
   │ Determine force output information corresponding to│
   │   walking environment of user in VR based on  │──── 1020
   │         received control information          │
   └─────────────────────────────────────────────┘
                             │
                             ▼
   ┌─────────────────────────────────────────────┐
   │        Output force feedback based on         │
   │      determined force output information       │──── 1030
   └─────────────────────────────────────────────┘
                             │
                             ▼
                      ╭─────────────╮
                      │     End     │
                      ╰─────────────╯
```

FIG.10

**Europäisches Patentamt**
**European Patent Office**
**Office européen des brevets**

# EUROPEAN SEARCH REPORT

Application Number

EP 20 20 8081

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2019/361527 A1 (YU ZUOXIN [CN] ET AL) 28 November 2019 (2019-11-28) * the whole document * ----- | 1-14 | INV. G06F3/01 G06F3/0481 A61H1/02 A61H3/00 B25J9/00 A63B24/00 |
| X | US 2019/046078 A1 (LIM BOKMAN [KR] ET AL) 14 February 2019 (2019-02-14) * the whole document * ----- | 1-14 | |

TECHNICAL FIELDS SEARCHED (IPC)

G06F
B25J
A61H
A63B

The present search report has been drawn up for all claims

| Place of search | Date of completion of the search | Examiner |
|---|---|---|
| Munich | 20 April 2021 | Dixon-Hundertpfund |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 20 20 8081

This annex lists the patent family members relating to the patent documents cited in the above-mentioned European search report.
The members are as contained in the European Patent Office EDP file on
The European Patent Office is in no way liable for these particulars which are merely given for the purpose of information.

20-04-2021

| Patent document cited in search report | | Publication date | Patent family member(s) | | Publication date |
|---|---|---|---|---|---|
| US 2019361527 | A1 | 28-11-2019 | CN  108619655  A | | 09-10-2018 |
| | | | US  2019361527  A1 | | 28-11-2019 |
| US 2019046078 | A1 | 14-02-2019 | NONE | | |

EPO FORM P0459

For more details about this annex : see Official Journal of the European Patent Office, No. 12/82